# EUROPEAN PATENT SPECIFICATION

(11) **EP 4 252 003 B1**
(45) Date of publication and mention of the grant of the patent: **05.03.2025**
(21) Application number: 21819229.2
(22) Date of filing: 30.11.2021
(51) Int. Cl.: G01N 33/561, G01N 33/569

(54) **ANALYTICAL METHOD FOR GLYCOCONJUGATES USING A CAPILLARY-BASED IMMUNOASSAY SYSTEM**
ANALYTISCHE METHODE FÜR GLYCOCONJUGATE MIT EINEM KAPILLARBASIERTEN IMMUNOASSAY-SYSTEM
MÉTHODE D'ANALYSE POUR LES GLYCOCONJUGATES UTILISANT UN SYSTÈME "IMMUNOASSAY" À BASE DE CAPILLAIRES

(30) Priority: 30.11.2020 EP 20210768
(43) Date of publication of application: 04.10.2023
(73) Proprietor: Janssen Pharmaceuticals, Inc., Titusville, NJ 08560 (US)
(72) Inventor: MANI, Jan, 3018 Bern (CH); KRIEFTEWIRTH, Michael, 3018 Bern (CH); REBER, Vera, 3018 Bern (CH); BIANCHI, Joëlle, 3018 Bern (CH); PIANTA, Annalisa, 3018 Bern (CH); CHAKKUMKAL, Anish, 2333 CN Leiden (NL); GRIJPSTRA, Jan, 2333 CN Leiden (NL); KAGABO, Diane, 2333 CN Leiden (NL)
(74) Representative: J A Kemp LLP
(86) International application number: PCT/IB2021/061115
(87) International publication number: WO 2022/113048

(56) References cited:
- MINSKER KEVIN ET AL: "Characterization of rVSV[Delta]G-ZEBOV-GP glycoproteins using automated capillary western blotting", VACCINE, ELSEVIER, AMSTERDAM, NL, vol. 38, no. 45, 18 September 2020 (2020-09-18), pages 7166 - 7174, XP086281921, ISSN: 0264-410X, [retrieved on 20200918], DOI: 10.1016/J.VACCINE.2020.08.001
- HAMM MELISSA ET AL: "Automated capillary Western dot blot method for the identity of a 15-valent pneumococcal conjugate vaccine", ANALYTICAL BIOCHEMISTRY, vol. 478, 1 June 2015 (2015-06-01), Amsterdam, NL, pages 33 - 39, XP055797812, ISSN: 0003-2697, DOI: 10.1016/j.ab.2015.03.021
- MARKELY LAM RAGA ANGGARA ET AL: "A high-throughput capillary isoelectric focusing immunoassay for fingerprinting protein sialylation", BIOTECHNOLOGY PROGRESS, vol. 32, no. 1, 8 December 2015 (2015-12-08), pages 235 - 241, XP055797777, ISSN: 8756-7938, DOI: 10.1002/btpr.2206
- DRIEDGER DARCY R. ET AL: "A Capillary Electrophoresis Laser-Induced Fluorescence Method for Analysis of Potato Glycoalkaloids Based on a Solution-Phase Immunoassay. 1. Separation and Quantification of Immunoassay Products", vol. 48, no. 4, 1 April 2000 (2000-04-01), pages 1135 - 1139, XP055797379, ISSN: 0021-8561, Retrieved from the Internet <URL:https://pubs.acs.org/doi/pdf/10.1021/jf990680t> DOI: 10.1021/jf990680t
- WANG JINYU ET AL: "Evaluation of automated Wes system as an analytical and characterization tool to support monoclonal antibody drug product development", JOURNAL OF PHARMACEUTICAL AND BIOMEDICAL ANALYSIS, ELSEVIER B.V, AMSTERDAM, NL, vol. 139, 21 December 2016 (2016-12-21), pages 263 - 268, XP029967499, ISSN: 0731-7085, DOI: 10.1016/J.JPBA.2016.12.024
- CASTLE ANDREW R. ET AL: "Application of high-throughput, capillary-based Western analysis to modulated cleavage of the cellular prion protein", JOURNAL OF BIOLOGICAL CHEMISTRY, vol. 294, no. 8, 1 February 2019 (2019-02-01), US, pages 2642 - 5291, XP055886369, ISSN: 0021-9258, Retrieved from the Internet <URL:https://www.jbc.org/article/S0021-9258(20)39364-9/pdf> DOI: 10.1074/jbc.RA118.006367

## Description

### Field of the Invention

The present invention relates to method for analysing a glycoconjugate in a test sample which comprises a mixture of at least 4 glycoconjugates, in particular to methods for analysing a glycoconjugate in a test sample comprising at least 4 glycoconjugates, in particular in a case where the glycoconjugate leads to the generation of broad signals which may comprise not fully-resolved peaks.

### Background

There is an increased pressure of regulatory authorities on biopharmaceutical manufacturers to demonstrate satisfactory programs for understanding, measuring, and controlling glycosylation in glycoconjugate-based drugs. However, the analysis of complex glycoconjugate compositions such as glycoconjugate vaccines, i.e. identification and quantification of individual glycoconjugates within such a composition, is a challenging task. Such analysis usually involves labor-intensive methods such as an enzyme-linked immunosorbent assay (ELISA) or a manual dot blot Western. Such a method typically involves a large amount of manual labor.

Furthermore, the frequently used ELISA provides no information on different glycosylation variants of each glycoconjugate.

A recent alternative is a capillary-based immunoassay method, i.e. a capillary western blot, which can be fullyautomated except for the sample preparation step [see e.g., Rustandi et al., Applications of an Automated and Quantitative CE-Based Size and Charge Western Blot for Therapeutic Proteins and Vaccines. 2016. In: Tran N., Taverna M. (eds) Capillary Electrophoresis of Proteins and Peptides: Methods and Protocols. Methods in Molecular Biology, vol 1466, pp. 197-217]. Such a method can significantly reduce the amount of manual labor as well as the overall analysis time.

In such methods, the different components of a sample are separated within a capillary and detected by means of a specific antibody.

Hamm et al. [Analytical Biochemistry 2015, 478: 33-39] describe the identification of individual glycoconjugates within a glycoconjugate vaccine composition using a capillary-based immunoassay method. However, they do not report the quantification of the glycoconjugates within said composition but merely suggest the possibility of performing a quantification. Hence the document also does not describe the measurement of calibration samples for the generation of a calibration curve. In order to achieve accurate quantification of all glycoconjugates within a glycoconjugate vaccine composition, it is mandatory to be able to accurately integrate the entire signal corresponding to each individual glycoconjugate. Thus this document only discloses an identity test for glycoconjugates. Accordingly it also does not disclose a computer program that allows quantification of glycoconjugates which lead to the generation of broad signals comprising not fully-resolved peaks. In addition, no analysis of bioconjugates is disclosed.

Minsker et al. [Vaccine 2020, 38: 7155-7174] describe the identification and relative quantification of glycoproteins using capillary western blot. In this method, the protein component of the glycoprotein is detected and the resulting signal is used to determine the relative abundance of the glycoproteins. Accordingly, this method does not include the measurement of calibration samples for the generation of a calibration curve nor absolute quantification of glycoproteins of interest. In addition, no details on the data analysis are provided. Instead, it is stated that "all unspecified settings were applied as default vendor recommendations".

Markely et al. [Biotechnology progress 2015, 32(1): 235-241] describe an isoelectric focusing immunoassay method for the relative quantification of different sialylated forms of a glycoprotein in order to monitor relative changes in sialylation during cell culturing. Accordingly, the document does not describe the generation of a calibration curve nor the absolute quantification of a glycoconjugate. Data analysis in this paper was performed using the Compass software according to the manufacturer's guidelines.

However, in general the quantification of analytes which lead to the generation of broad signals, in particular broad signals comprising not fully-resolved peaks, remains a challenge [see e.g., Fig. 5D in Castle et al., J. Biol. Chem. 2019, 294(8):2642-2650].

Whereas some glycoconjugates may lead to the generation of narrow signals which are quantifiable by currently available capillary-based immunoassay methods, others do not. For example, a glycoconjugate comprising an EPA carrier protein with four glycosylation sites, can exist in a mono, di-, tri- or tetraglycosylated form. Such a glycoconjugate can be produced by enzymatic conjugation of the polysaccharide component to a carrier protein, e.g. using the PglB oligosaccharyltransferase [see e.g. WO 2015/124769; WO 2020/191082; Poolman and Wacker, J. Infect. Dis. (2016) v.213(1), pp. 6-13 and references therein]. In such case, i.e. enzymatic conjugation of a polysaccharide component to a carrier protein in a cell, the glycoconjugate is also referred to as a bioconjugate.

The signals generated for such bioconjugates upon analysis by means of a capillary-based immunoassay method are typically broad and comprise several peaks corresponding to different glycosylation states, e.g. a mixture of mono-, di-, tri- or tetraglycosylated forms of the bioconjugate that are typically not fully-resolved, i.e. not baseline separated.

In such a case, the currently available capillary based immunoassay system, namely the Wes^{™} system in combination with the software "Compass for SW version 3.1.7" [commercially available from Bio-techne; https://www.proteinsimple.com/wes.html, accessed on 07 September 2020], fails to provide reliable quantitative data on the test sample.

Hence, known capillary based immunoassay methods are not suitable for the identification and absolute quantification of all glycoconjugates, in particular glycoconjugates which result in broad signals as it is typically the case for bioconjugates. Such broad signals may span a molecular weight range of between 100 - 500 kDa, typically of between 200 - 400 kDa. Examples thereof are bioconjugates comprising a carrier protein with a defined number of glycosylation sites, e.g. 1-10 such as 4, and where the glycans are conjugated to a limited number of specific glycosylation sites, e.g. 1-10 such as 1, 2, 3, or 4. In particular, this is the case for bioconjugates and mixtures thereof comprising:
- an EPA carrier protein with four glycosylation sites and
- a polysaccharide component corresponding to different *Escherichia coli* O-antigens.

As described above, such a bioconjugate where the glycans are coupled to a limited number of specific glycosylation sites, such as 4, can be produced by enzymatic conjugation of the polysaccharide component to a carrier protein using the PglB oligosaccharyltransferase [e.g. WO 2015/124769; WO 2020/191082; Poolman and Wacker, J. Infect. Dis. (2016) v.213(1), pp. 6-13 and references therein].

Furthermore, the identification and absolute quantification of closely-related glycoconjugates, i.e. glycoconjugates which only differ in the PS component corresponding to different serotypes of an antigen, is a unique challenge for each glycoconjugate, particularly bioconjugates. Such analysis by means of a capillary-based immunoassay method has not yet been described for the glycoconjugates described above.

The following documents are also mentioned:
- Minsker et al (2020) Vaccine, 38(45): 7166-7174 which is concerned with the characterization of rVSV [Delta] G-ZEBOV-GP glycoproteins using automated capillary western blotting;
- Hamm et al (2015) Analytical Biochemistry, 478: 33-39 which is concerned with automated capillary western dot blot method for the identity of a 15-valent pneumococcal conjugate vaccine;
- Markely et al (2015) Biotechnology progress, 32(1): 235-241 which is concerned with a high-throughput capillary isoelectric focussing immunoassay for fingerprinting protein sialylation;
- Driedger et al (2000) Journal of Agricultural and Food Chemistry, 48(4): 1135-1139 which is concerned with a capillary electrophoresis laser-induced fluorescence method for analysis of potato glycoalkaloids;
- Wang et al (2016) Journal of Pharmaceutical and Biomedical Analysis, 139: 263-268 which is concerned with evaluation of automated Wes system as an analytical and characterization tool to support monoclonal antibody drug product development; and
- Andrew et al (2019) Journal of Biological Chemistry, 294(8): 2642-5291 which is concerned with application of high-throughput, capillary based Western analysis to modulated cleavage of the cellular prior protein.

The aim of the present invention is thus to mitigate these limitations of the prior art.

### Summary of the Invention

The present invention provides method for analysing a glycoconjugate in a test sample which comprises a mixture of at least 4 glycoconjugates,
wherein the analysis includes:
   - the identification of said glycoconjugate and
   - the absolute quantification of said glycoconjugate based on a calibration curve;
wherein the method comprises the steps of:
   (a) providing the test sample and a set of calibration samples, optionally a ladder sample, and optionally a control sample;
   (b) measuring, by means of a capillary-based immunoassay system, in individual capillaries: said test sample, said calibration samples, optionally said ladder sample, and optionally said control sample, thereby generating a dataset for each capillary; and
   (c) analysing said dataset by means of a computer program which provides at least the following functions:
      - receiving limits for integration; and
      - calculating a background signal for each capillary; and
      - subtracting a background signal from a signal generated in each capillary.

### Brief description of the drawings

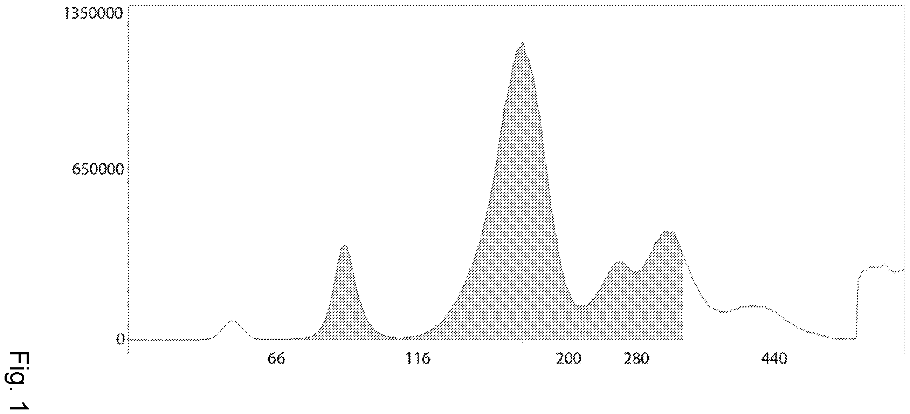

Figure 1: Analysis of an electropherogram resulting from the measurement of a glycoconjugate comprising an EPA carrier protein and a PS component corresponding to an *E. coli* O-antigen (serotype O6), as described in example 1, using the Dropped Lines function of the software "Compass for SW version 3.1.7" that is included in the WES^{™} system. This type of area calculation is also referred to as perpendicular drop method. Analysis using the Compass software fails to integrate the whole signal area (only the marked area was integrated by the Compass software). The Y-axis represents the chemiluminescent signal intensity and the X-axis represents the molecular weight (MW) expressed in kDa.

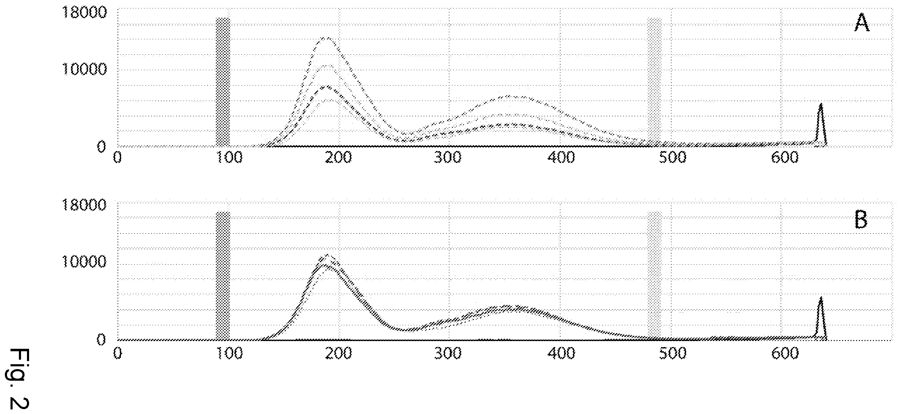

Figure 2: Integration of electropherograms resulting from the analysis of a glycoconjugate comprising an EPA carrier protein and a PS component corresponding to an *E. coli* O-antigen (serotype O4), as described in example 1, using the computer program as described herein. The Y-axis represents the chemiluminescent signal intensity and the X-axis represents the molecular weight (MW) expressed in kDa. Measurements were performed using the WES^{™} system. The integration range is indicated by two vertical rectangles at 100 and 480 kDa. The width of the rectangle represents the molecular weight window to calculate the background signal for a specific capillary. The background signal is automatically subtracted from the signal resulting from the analyte within the corresponding capillary. The integration range and the width of the molecular weight window to calculate the capillary-specific background signal can be adjusted by the user.
   A) Overlay of electropherograms resulting from the analysis of four calibration samples (one replicate shown). This is used to generate the calibration curve.
   B) Overlay of 4 replicates of the analysis of the above glycoconjugate in a multivalent glycoconjugate vaccine composition comprising 10 glycoconjugates. This confirms the technical reproducibility of the determination of identity and absolute quantification of the analyte.

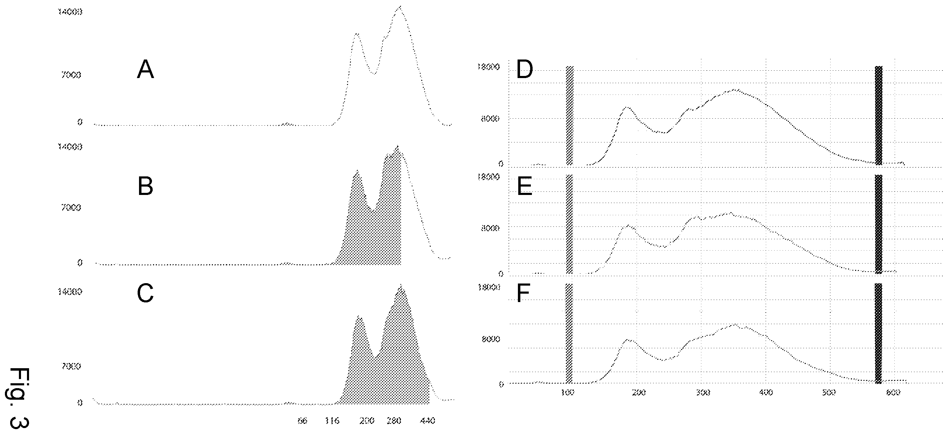

Figure 3: Analysis of electropherograms resulting from three measurements (triplicates) of an O25B O-EPA bioconjugate using the WES^{™} system. The Y-axis represents the chemiluminescent signal intensity and the X-axis represents the molecular weight (MW) expressed in kDa.

A-C: Integration of O25B-EPA triplicate measurements using the Dropped Lines function of the software "Compass for SW version 3.1.8" that is included in the WES^{™} system, also referred to as perpendicular drop method. Analysis using the Compass software leads to different integration ranges (marked area) for each measurement. In each case the Compass software fails to integrate the whole area of the signal.

D-F: Integration of the same O25B O-EPA triplicate measurements as in A-C but integration performed using the computer program as described herein. The integration range is indicated by two vertical rectangles at 100 and 570 kDa. The width of the rectangle represents the molecular weight window to calculate the background signal for a specific capillary. The background signal is automatically subtracted from the signal resulting from the analyte within the corresponding capillary. Integration of the whole area of the signal is performed in a reproducible manner.

### Detailed description of the invention

Unless otherwise stated, the following definitions shall apply in this specification:
As used herein, the term "a", "an", "the" and similar terms used in the context of the present invention (especially in the context of the claims) are to be construed to cover both the singular and plural unless otherwise indicated herein or clearly contradicted by the context.

As used herein, the terms "including", "containing" and "comprising" are used herein in their open, non-limiting sense. It is understood that the various embodiments, preferences and ranges may be combined at will.

**Glycoconjugate:** The term "glycoconjugate" is known in the field and particularly describes chemical entities covalently bound to one or more polysaccharide (s) (PS). Such glycoconjugate may be obtained by biological conjugation in a living cell ("bioconjugate" or "biological conjugate") or may be obtained by chemical conjugation of a polysaccharide ("chemical" or "synthetic" glycoconjugate). Suitable chemical entities include proteins/peptides and lipids, the corresponding glycoconjugates being glycoproteins and glycolipids. Particularly suitable, within the scope of the invention, are glycoconjugates selected from the group of glycoproteins. As described above, identification and absolute quantification of glycoconjugates that are bioconjugates (i.e. a subgroup of glycoproteins) is particularly challenging using the methods of the prior art. However, bioconjugates are particularly suitable glycoconjugates in the context of the present invention.

In more detail, the term glycoprotein includes "traditional glycoproteins" and "glycoconjugate vaccines".

In traditional glycoproteins, the emphasis is on the protein part, such as for instance for antibodies or erythropoietin where the 'active' principle is more residing in the protein part, and the glycans play a role for instance in half-life or defining other properties. Such traditional glycoproteins find widespread use in pharmaceutical applications, such as in oncology or inflammatory diseases.

In glycoconjugate vaccines, the emphasis is on the glycan part, to which an immune response is desired because the glycans are the relevant antigens, and the protein part merely serves as a carrier to lead to a desired T-cell memory immune response. Accordingly, glycoconjugate vaccines differ from the above described "traditional glycoproteins".

In preferred embodiments, the glycoconjugates are glycoconjugate vaccines that are part of a glycoconjugate vaccine composition. Glycoconjugate vaccines comprise a carrier protein which is linked to one or more PS components, said PS components corresponding to an antigen, in particular a bacterial O-antigen. Bioconjugates, as opposed to chemical glycoconjugates, have recently emerged as particularly suitable glycoconjugate vaccines. Particularly suitable within the scope of the invention are glycoproteins selected from the group of glycoconjugate vaccines, more particularly bioconjugates.

Bioconjugate: The term is discussed above. Specifically, a bioconjugate is a glycoconjugate prepared in a host cell, wherein the host cell machinery produces the glycan and the carrier protein and links the glycan to the carrier protein, e.g., via N-links of asparagine or arginine. A particularly preferred host cell for producing bioconjugates is *E. coli,* preferably comprising nucleic acid encoding: (i) the carrier protein, (ii) an oligosaccharyltransferase such as *C*. *jejuni* PglB that is capable of covalently linking O-antigen polysaccharides to an asparagine (Asn) residue in a glycosylation consensus sequence (Asn-X-Ser (Thr), wherein X can be any amino acid except Pro) in a carrier protein via N-linked glycosylation, and (iii) an *rfb* gene cluster encoding the enzymes responsible for generating the O-antigen polysaccharide of a desired serotype. By creating host cells with a different *rfb* locus, different bioconjugates can be prepared, e.g. comprising O-antigen polysaccharides from different *E. coli* or *Shigella* serotypes. Culturing such host cells will produce the bioconjugates comprising the carrier protein to which the O-antigen polysaccharide encoded by the *rfb* locus is covalently attached, within the periplasm of the host cell. A more detailed description for production of bioconjugates in such host cells can for instance be found in WO 2009/104074, WO 2015/124769, WO 2017/035181, or WO 2020/191082. Optimized variants of the PglB oligosaccharyltransferase for production of bioconjugates of specific *E. coli* O-antigens have been described in WO 2020/191088. The present invention deals with novel and improved methods of identification and absolute quantification of the produced bioconjugates from such host cells. The host cell for production of bioconjugates is typically a bacterial cell, preferably a gram-negative bacterial cell, and in preferred embodiments the host cell is *E. coli.*

Particularly useful bioconjugates include carrier proteins to which one or more polysaccharides are attached. Such bioconjugates are for instance used as the active components of certain vaccines, which aim at inducing functional immune responses against the polysaccharides of the bioconjugates. In embodiments of the invention, said bioconjugate comprises one carrier protein and one or more polysaccharides covalently bound to said carrier protein, preferably 1 to 4 polysaccharides covalently bound to said carrier protein.

In embodiments of the invention, the bioconjugate is a conjugation product containing an *E. coli* O-antigen polysaccharide covalently bound to a carrier protein. In embodiments of the invention, the bioconjugate is a conjugation product containing a *Shigella* O-antigen polysaccharide covalently bound to a carrier protein.

The term O-antigen is known in the field and used in its normal context, it is not to be confused with O-linked. In typical embodiments, the O-antigen polysaccharide is N-linked to the carrier protein. The term O-antigen polysaccharide generally refers to a repetitive glycan polymer contained within an LPS of a bacteria, such as *E. coli.* The O-antigen of *E. coli* is a polymer of immunogenic repeating oligosaccharides (typically 1-40 repeating units, e.g. 5-30 repeating units) and typically used for serotyping and glycoconjugate vaccine production.

**Carrier protein:** The term is discussed above. In particular embodiments of the invention, the carrier protein is a detoxified Exotoxin A of *Pseudomonas aeruginosa* (EPA; the terms Exotoxin A and ExoProtein A of *P. aeruginosa,* or EPA, are used interchangeably). In such embodiments, the EPA preferably comprises 1 to 10, preferably 2 to 4, glycosylation sites.

In a particular embodiment, the EPA comprises four glycosylation sites. In a particular embodiment, the EPA comprises four glycosylation sites having SEQ ID NO 1, preferably having SEQ ID NO 2. See for example WO 2015/124769, WO 2017/035181, or WO 2020/191082 for a description of examples of bioconjugation of various *E.coli* O-antigen polysaccharides to EPA carrier protein, and a representative amino acid sequence of EPA carrier protein. See for example WO 2009/104074 for a description of examples of bioconjugation of *Shigella* O-antigen polysaccharides to EPA carrier protein.

For EPA, various detoxified protein variants have been described in literature and could be used as carrier proteins. For example, detoxification can be achieved by mutating and deleting the catalytically essential residues L552V and ΔE553.

In one non-limiting preferred embodiment, the carrier protein of a bioconjugate according to the invention comprises SEQ ID NO: 3.

A glycoconjugate, in particular a bioconjugate, comprising an EPA carrier protein with four glycosylation sites can exist in a mono-, di-, tri- or tetraglycosylated form.

**Polysaccharide:** The term "polysaccharide" is known in the field and particularly describes polymeric carbohydrates composed of monosaccharide units bound together by glycosidic linkages, either linear or branched. Such polysaccharides are characterized by their repeating units, each repeating unit described with their respective monosaccharide composition. Said repeating units include one or more monosaccharides which can also be chemically modified (e.g. aminated, amidated, sulphonated, acetylated, phosphorylated, etc). Typically found monosaccharides in said repeating units are cyclic or linear monosaccharides containing three to seven carbon atoms. In the specific case of glycoconjugate vaccines, the conjugated polysaccharide originates from a pathogenic species (e.g. *Escherichia coli*) with said repeating unit defined by the genetics of the specific pathogen. The repeating unit can thus be a specific marker / identifier of the pathogen.

The term "polysaccharide component" consequently denotes one or more glycan chain(s) of a glycoconjugate. Glycans can be monomers or polymers of sugar residues, but typically contain at least three sugars, and can be linear or branched. A glycan may include natural sugar residues (e.g., glucose, N- acetylglucosamine, N-acetyl neuraminic acid, galactose, mannose, fucose, arabinose, ribose, xylose, etc.) and/or modified sugars (e.g., 2'-fluororibose, 2'-deoxyribose, phosphomannose, 6'-sulfo N-acetylglucosamine, etc). The term "glycan" includes homo- and heteropolymers of sugar residues. The term "glycan" also encompasses a glycan component of a glycoconjugate (e.g., of a glycoprotein, glycopeptide, glycolipid). The term also encompasses free glycans, including glycans that have been cleaved or otherwise released from a glycoconjugate.

The term "O-acetylated polysaccharide", as used herein, refers to polysaccharides where one or more monosaccharides of the repeating unit are chemically modified by acetylation. Said monosaccharides have one or more of their present hydroxyl groups acetylated. For pathogen-derived repeating units used in glycoconjugate vaccines, the O-acetylation of certain monosaccharides can be essential to induce an immune response for said pathogen. Non-limiting examples of pathogen-derived polysaccharide components are shown in Table 1.

The term "serotype" as used herein, refers to glycoconjugates having different polysaccharide chains which are derived from different bacterial serotypes. Examples of glycans from a number of *E. coli* serotypes are identified below in Table 1.

In particular embodiments, the PS component of the glycoconjugate corresponds to different serotypes of the "O-antigen" of *Escherichia coli (E. coli).*

The "O-antigen" is part of the bacterial Lipopolysaccharide (LPS). LPS consist of a lipid and a PS component, wherein the PS component is further divided into a core structure and the "O-antigen". In addition, each O-antigen is composed of n repeating units, wherein n is 1-100, such as 1-50, 1-40, 1-30, 1-20, 1-10, 3-50, 3-40, e.g. at least 5, such as 5-40, 5-30, e.g. 7-30, e.g. 7 to 25, e.g. 10 to 20, e.g. 5 - 20, repeating units. Each repeating unit comprises non-modified and/or modified monosaccharides.

The term "modified mono-saccharides" in non-limiting embodiments includes N-acetylation, O-acetylation, amidation and/or amination of mono-saccharides. Such modified monosaccharides may comprise one or more modifications, particularly one, two or three of the above modifications, at the same mono-saccharide.

In particular embodiments, modified monosaccharides are O-acetylated and/or N-acetylated monosaccharides, specifically monosaccharides comprising one O-acetylation or N-acetylation.

In embodiments of the invention, suitable repeating units comprise monosaccharides selected from the group consisting of Mannose, Rhamnose, Glucose, Fucose, Galactose, modified Mannose, modified Rhamnose, modified Glucose, modified Fucose, and modified Galactose.

Non-limiting and exemplary structures of *E. coli* O-antigen polysaccharides are shown below in Table 1. A single repeating unit for each *E. coli* O-antigen polysaccharide is shown. In this table, each n is independently an integer of 1 to 100, such as 1-50, 1-40, 1-30, 1-20, 1-10, 3-50, 3-40, 5-30, e.g. at least 5, such as 5-40, e.g. 7-30, e.g. 7 to 25, e.g. 10 to 20, e.g. 5 - 20, but in some instances can be 1-2.

**Table 1: Structures of E. coli O-antigen polysaccharides**

| ***E. coli* O-antigen Polysaccharide** |
|---|
| **Structure of Repeating Unit** |
| O1A antigen polysaccharide (O1A) |
| 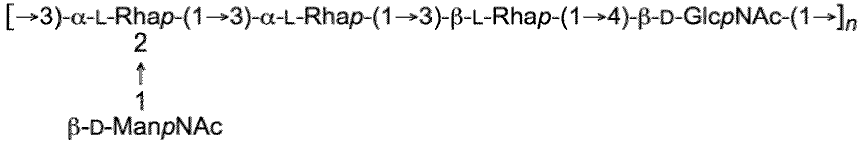 |
| O2 antigen polysaccharide (O2) |
| 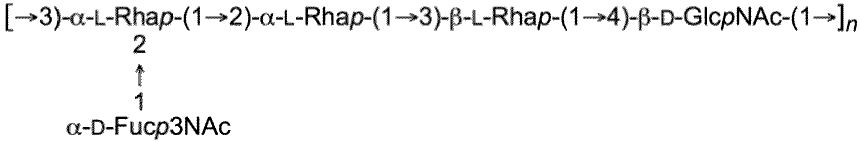 |
| O4 antigen polysaccharide (O4) |
| 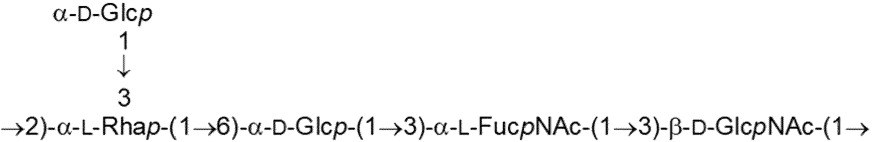 |
| O6A antigen polysaccharide (O6) |
| 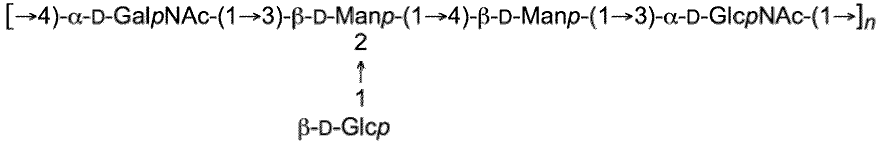 |
| O8 antigen polysaccharide (O8) |
| **α-D-Manp3Me-(1→[3)-β-D-Manp-(1→2)-α-D-Manp-(1→2)-α-D-Manp-(1→]n** |
| O15 antigen polysaccharide (015) |
| **[→2)-β-D-Galp-(1→3)-α-L-FucpNAc-(1→3)-β-D-GlcpNAc-(1→]ₙ** |
| O16 antigen polysaccharide (016) |
| 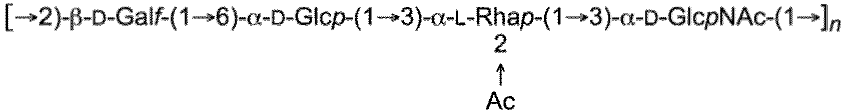 |
| O18A antigen polysaccharide (O18A) |
| 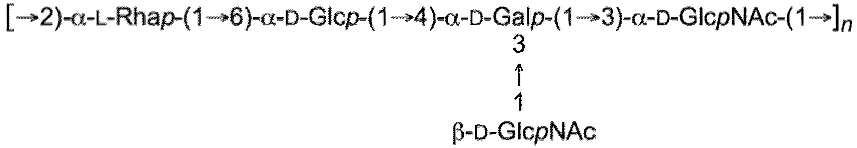 |
| O25B antigen polysaccharide (O25B) |
| 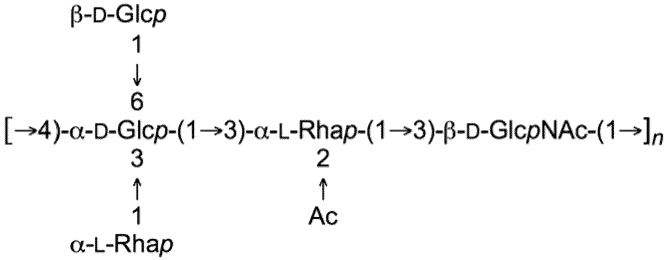 |
| O75 antigen polysaccharide (O75) |
| 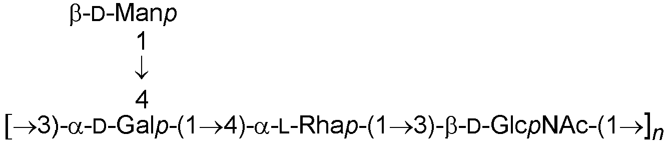 |

The various serotypes of *E.coli* differ in the sugar composition of the O-antigen. However, within the same serotype classification, the O-antigen may vary in the number of repeating units and the degree of acetylation.

**Sample:** The term sample is known in the field. It includes any material which, optionally after dilution, may be supplied to an analytical system. As used herein, the term "test sample" relates to the sample to be analysed. The test sample comprises at least one glycoconjugate, preferably bioconjugate, along with other components, typically a mixture of at least four glycoconjugates, preferably bioconjugates, and one or more other components. Such samples particularly include (i) production batches of glycoconjugates (including in-process batches and released/stored production batches); (ii) compositions comprising multiple glycoconjugates, such as pharmaceutical compositions comprising a multivalent vaccine.

Suitable test samples comprise, in addition to the glycoconjugate(s), (i) an aqueous matrix; (ii) optionally polysaccharides not bound to carrier protein (herein: "free PS"); (iii) optionally non-related proteins; (iv) optionally carrier protein free of polysaccharides. The aqueous matrix (i) may contain one or more of buffers (e.g. phosphate buffer), inorganic salts (e.g. NaCl), sugar alcohols (e.g. D-Sorbitol), non-ionic surfactants (e.g. Polysorbate 80). The non-related proteins (iii) may include up to 10% process related impurities (e.g. host cell proteins). In an embodiment, the test sample comprises at least 4 and up to 20 glycoconjugates, e.g. at least 4 and up to 12 glycoconjugates, e.g. 4, 5, 6, 7, 8, 9, 10, 11, or 12 glycoconjugates. In certain embodiments, the test sample comprises 4, 9 or 10 glycoconjugates.

The term "calibration sample" relates to a sample comprising a known concentration of the glycoconjugate to be analysed. A set of calibration samples thus relates to a multitude of calibration samples with graded concentrations of the glycoconjugate. The concentration range of the set of calibration samples also covers the expected concentration of the glycoconjugate within the test sample. Such a set of calibration samples is suitable for establishing a calibration curve for the absolute quantification of the glycoconjugate within the test sample.

The term "control sample" relates to a sample comprising a known concentration of the glycoconjugate to be analysed for verification of the suitability of the capillary-based immunoassay system to perform the intended analysis. Such control samples are also known as system suitability controls (SSC). Such control samples may comprise additional components such as a suitable buffer (e.g. the same buffer as the test sample) but comprise only one glycoconjugate and therefore typically differ from the test sample. Typically, the concentration of the glycoconjugate in the control sample is higher than in the test sample.

The term "ladder sample", or simply "ladder", relates to a sample comprising a size standard. Such size standards are known and typically include a mixture of proteins or modified proteins of a known molecular weight. A suitable ladder sample comprises a mixture of biotinylated proteins with molecular weights spanning the range from 66 kDa to 440 kDa.

**Capillary-based immunoassay:** The term immunoassay generally refers to an analysis method in which an antibody is used for the identification and/or quantification of specific components within a sample. In case the assay is performed in a capillary, the method is referred to as a capillary-based immunoassay. Detailed information may be taken e.g. from Moser et al., Electrophoresis 2008, 29(16): 3279-3295. Alternatively, detection by an antibody may be replaced by an aptamer. Within the scope of the invention, a capillary-based immunoassay refers to an automated, capillary-based western blot. Systems for performing such capillary-based western blots are known in the field and commercially available. A particular example is the Wes^{™} system from Bio-Techne, c.f. https://www.proteinsimple.com/wes.html, accessed on 07 September 2020. This system has been used for the analysis of several glycoconjugates [e.g., Rustandi et al., Applications of an Automated and Quantitative CE-Based Size and Charge Western Blot for Therapeutic Proteins and Vaccines. In: Tran N., Taverna M. (eds) Capillary Electrophoresis of Proteins and Peptides. Methods in Molecular Biology, vol 1466]. However, currently available methods are not suitable to achieve the aim of the present invention, which is the identification and absolute quantification of glycoconjugates, particularly bioconjugates, including those which lead to the generation of broad signals comprising not fully-resolved peaks. As discussed above, an example of such a glycoconjugate is a glycoconjugate comprising an EPA carrier protein with four glycosylation sites and a PS component corresponding to an *E. coli* O-antigen as listed in claim 5 (ii).

**Identification and/or absolute quantification of a glycoconjugate:** As discussed above, analysis includes identification and absolute quantification of a glycoconjugate, in particular a bioconjugate. In principle, both, identification and quantification of a glycoconjugate can either relate to the glycoconjugate as a whole or the individual components of said glycoconjugate, i.e. the carrier protein and/or the PS component. The decisive aspect is the choice of the primary antibody which is applied to detect the glycoconjugate (e.g. the primary antibody may recognize the PS component or the carrier protein). The constant region (Fc) of the primary antibody can then be recognized by a secondary antibody which is capable of generating a detectable signal which can be used for absolute quantification of the glycoconjugate based on a calibration curve.

In the context of this invention, in particular the identification and absolute quantification of the PS component is of relevance. Therefore, a primary antibody is used which specifically binds to the PS component of the glycoconjugate. Hence, in a preferred embodiment, identification and absolute quantification of the glycoconjugate refers to identification and absolute quantification of the PS component of the glycoconjugate.

Thus, the concentration of the glycoconjugate, as mentioned in step (a1) below, refers to the concentration of the PS component. For example, a glycoconjugate concentration of 0.100 µg mL⁻¹ refers to a concentration of 0.100 µg mL⁻¹ of the PS component, independent of the amount of carrier protein within the sample (as mentioned above, one or more polysaccharides are covalently bound to the carrier protein). Nevertheless, identification and absolute quantification of the glycoconjugate with respect to the carrier protein is also possible. In this case a primary antibody can be used which specifically binds to said carrier protein.

Throughout this specification a number of **abbreviations** are used, including:
- *E. coli*: *Escherichia coli*
- EPA: detoxified Exotoxin A of *P. aeruginosa*
- Fc: constant region of an antibody
- HRP: horseradish peroxidase
- LPS: lipopolysaccharide
- PS: polysaccharide
- SSC: system suitability control

The above abbreviations are common in the field.

The invention relates to a method for analysing a glycoconjugate in a test sample which comprises a mixture of at least 4 glycoconjugates, wherein the analysis includes both, the identification of said glycoconjugate and the quantification of said glycoconjugate. The inventive method comprises the steps of:
(a) providing the test sample and a set of calibration samples for establishing a calibration curve, optionally a ladder sample, and optionally a control sample;
(b) measuring, by means of a capillary-based immunoassay system, in individual capillaries: said test sample, said calibration samples, optionally said ladder sample, and optionally said control sample, thereby generating a dataset for each capillary; and
(c) analysing said dataset by means of a computer program which provides at least the following functions:
   - receiving limits for integration; and
   - calculating a background signal for each capillary; and
   - subtracting a background signal from a signal generated in each capillary.

More precisely, the term "identification" relates to the identification of a glycoconjugate based on the PS component of said glycoconjugate and the term "quantification" relates to the absolute quantification of said glycoconjugate based on a calibration curve.

The term "receiving limits", as used herein, relates to "receiving molecular weight range limits", i.e. receiving limits for a molecular weight range in which a signal is integrated. As outlined above, such limits for integration may for instance span a molecular weight range of between 100 - 500 kDa (e.g. limits for integration being 100 kDa and 500 kDa), typically of between 200 - 400 kDa. This method shall be explained in further detail below, first referring to suitable samples followed by the individual method steps:

**Test Sample:** A test sample of present invention comprises a mixture of at least 4 glycoconjugates. A broad range of samples may be used in the context of this invention. Quantifiable glycoconjugates also include glycoconjugates which lead to the generation of broad signals comprising not fully-resolved peaks and are hence not quantifiable by currently available capillary-based immunoassay methods. Such broad signals may for example span a molecular weight range of between 50-600 kDa, preferably of between 100 - 500 kDa, typically of between 200 - 400 kDa. Examples of such broad signals are shown in Fig. 1 - 3. For instance, the signal shown in Fig. 1 spans a molecular weight range of approximately 370 kDa, i.e. the signal starts at approximately 80 kDa and finishes at approximately 450 kDa. In this case the Compass Software included in the WES^{™} system fails to reliably integrate the whole signal area. Such analysis methods may also be applied to a mixture of closely related glycoconjugates, i.e. glycoconjugates, particularly bioconjugates, which only differ in the polysaccharide component.

In one embodiment, the glycoconjugate comprises one carrier protein and one or more polysaccharides covalently bound to said carrier protein, preferably wherein said carrier protein is a detoxified Exotoxin A of *Pseudomonas aeruginosa* (EPA) with four glycosylation sites.

In one embodiment, the glycoconjugate comprises one carrier protein and one or more polysaccharides covalently bound to said carrier protein, wherein said polysaccharide is an *E. coli* O-antigen, preferably selected from the group consisting of O1A, O2, O4, O6A, O8, 015, 016, O18A, O25B or O75 antigen.

In one embodiment, the glycoconjugate comprises one carrier protein and one or more polysaccharides covalently bound to said carrier protein, wherein said glycoconjugate is produced by enzymatic conjugation of the PS component to the carrier protein using an oligosaccharyltransferase (such as PglB) in *E. coli.* The glycoconjugates thereby are referred to as 'bioconjugates' and differ in this embodiment from glycoconjugates where the carrier protein and the PS component are chemically coupled.

In certain embodiments, said polysaccharide(s) comprise 1-100, preferably 3-30, more preferably 5 - 20, repeating units. Said repeating units comprise non-modified monosaccharides and/or modified monosaccharides. In particular, the modified monosaccharides are O-acetylated and/or N-acetylated monosaccharides, e.g. monosaccharides comprising one O-acetylation or N-acetylation.

The test sample may comprise, and typically does, additional components. In one embodiment, the test sample further comprises an aqueous matrix, which optionally includes one or more of buffers, inorganic salts, sugar alcohols, and/or non-ionic surfactants. Optionally, the sample further comprises polysaccharides not bound to carrier protein ("free PS"). Optionally, the sample further comprises non-related proteins, for example host-cell proteins. Optionally, the sample further comprises a carrier protein free of polysaccharides.

In certain embodiments, the test sample comprises a multitude of different glycoconjugates, preferably 4-20, e.g. 4 - 10 glycoconjugates. In a specific embodiment, the sample comprises 4, 9 or 10 glycoconjugates, said glycoconjugate differing in the *Escherichia coli* polysaccharide components listed above.

In a preferred embodiment, the test sample is a multivalent vaccine composition adapted for direct use in a subject in need thereof.

Given the above, the inventive method also allows the identification and/or quantification of mono-, di-, tri- or tetraglycosylated variants of said glycoconjugate and mixtures thereof by means of the capillary based immunoassay method described herein. Thus, the invention also provides for a method, wherein said analysis includes the identification of mono-, di-, tri- and/or tetraglycosylated variants of said glycoconjugate and mixtures thereof and/or the quantification of mono-, di-, tri- and/or tetraglycosylated variants of said glycoconjugate and mixtures thereof.

As described above, the term "identification" more precisely relates to the identification of a glycoconjugate based on the PS component of said glycoconjugate and the term "quantification" more precisely relates to the absolute quantification of mono-, di-, tri- and/or tetraglycosylated variants of said glycoconjugate based on a calibration curve.

**Step a:** Providing samples to an analytical device is known per se. The concentration of a glycoconjugate in a test sample may vary over a broad range, if required it is adapted in step (a1), outlined below. In a preferred embodiment, step (a) further comprises one or more of the following steps (a1), (a2) and /or (a3), preferably in the order as indicated:
In one embodiment, this includes (a1) adjusting the concentration of the glycoconjugate to an expected concentration of 0.01 - 0.50 µg mL⁻¹. As outlined above, the concentration of the glycoconjugate relates to the concentration of the PS component of the glycoconjugate. It will be clear to the skilled person that this step is optional, e.g. if the concentration of the glycoconjugate is already expected to be in this range, this step is not needed.

In one embodiment, this includes (a2) adding to the sample one or more auxiliary reagents selected from a sample buffer, a disulfide bridge reducing agent, such as dithiothreitol, and one or more markers, such as fluorescent protein markers with a known molecular weight. Said markers can be used as a molecular weight reference within each capillary and are used to account for differences in the electrophoretic migration between individual capillaries.

In one embodiment, this includes (a3) applying a heat program and thereby denaturing the sample. An example of a suitable heat program is heating the respective sample at 95°C for 5 min.

**Step b:** Measuring a sample by means of a capillary-based immunoassay method is known per se. In a preferred embodiment, step (b) further comprises one or more of the following steps (b1) to (b7), preferably in the order as indicated:
In one embodiment, this includes (b1) loading an analysis matrix into the capillaries of said immunoassay system; preferably the analysis matrix is a size-exclusion matrix; preferably said size-exclusion matrix comprises at least two matrix components with different pore sizes (stacking and separation matrix).

In a preferred embodiment, glycoconjugates are separated based on their size / molecular weight. Thus, in a preferred embodiment, identification and absolute quantification of a glycoconjugate by means of an immunoassay system relates to identification and absolute quantification of a glycoconjugate by means of a size-based / molecular weight based immunoassay system. Analysis of glycoconjugates based on their size / molecular weight differs from analysis of glycoconjugates based on their isoelectric point (e.g. isoelectric focusing immunoassay).

In one embodiment, this includes (b2) loading the test sample, calibration samples, optional ladder sample, and optional control sample into the capillaries of said immunoassay system.

In one embodiment, this includes (b3) separating the components of said samples, preferably according to the molecular weight of each component. This separation is performed in the analysis matrix in the capillaries of said immunoassay system.

In one embodiment, this includes (b4) immobilising the components of said sample, e.g. by photochemical crosslinking.

In one embodiment, this includes (b5) applying a glycan-specific primary antibody which binds to said glycoconjugate. Glycan-specific antibodies bind to the PS part of the glycoconjugate, and are specific for a given PS structure. They are known as such and can be obtained from commercial sources or prepared by known standard techniques. They may be monoclonal or polyclonal antibodies.

In one embodiment, this includes (b6) applying a secondary antibody which binds to the primary antibody and which generates a detectable signal. The secondary antibody may for instance bind to the constant region (Fc) of the primary antibody. A variety of possibilities to generate said detectable signal (step b6) are well-known to a person skilled in the art. For example, said signal can be generated by applying in step (b6) a secondary antibody which is covalently linked to an enzyme, which is capable of catalyzing a chemiluminescent reaction, a chemifluorescent reaction or a chemical reaction leading to a colored or fluorescent product. Examples of such enzymes are horseradish peroxidase or alkaline phosphatase.

It is however clear to the person skilled in the art that a detectable signal can also be generated by other means. For example, said secondary antibody (step b6) can be linked to a fluorescent dye, such as an Alexa Fluor, Alexa Fluor Plus, IRDye, fluorescein isothiocyanate (FITC), Cy3, Cy5, allophycocyanin (APC), tetramethylrhodamine, DyLight, Texas Red, Texas Red-X, phycoerythrin (R-PE), Qdot, 3-Car-boxy-6,8-difluor-7-hydroxycumarin or pacific orange dye. Another possibility is to apply a primary antibody in step (b5), which is linked to biotin. In this case, a detectable signal may be generated by in step (b6) applying a streptavidin protein which is linked to HRP instead of applying a secondary antibody. As described above, in step (b7), a substrate for HRP is applied.

Another possibility is in step (b6) to apply a secondary antibody which is linked to a gold nanoparticle.

In one embodiment, this includes (b7), in case the secondary antibody is linked to an enzyme, applying a substrate for said enzyme. In a preferred embodiment, said secondary antibody is linked to the enzyme horseradish peroxidase (HRP). The detectable signal is generated by applying a substrate in step (b7). In a preferred embodiment, the substrate is a luminol/peroxide mixture.

**Step c:** The analysis of data by a computer program is known. However, as discussed above and shown in Fig. 1, 3 and Tables 3 - 5, the present standard method of analysis of data is insufficient and does not allow reliable quantification.

By following the above protocol, this shortcoming is resolved. In a preferred embodiment, step c) further comprises one or more of the following steps, preferably in the order as indicated:
(c1) receiving the dataset for every capillary generated in step b);
(c2) identifying the background for every capillary;
(c3) subtracting the individual background signal from the measured signal of the corresponding capillary;
(c4) obtaining an area under the curve for every capillary by integrating the background corrected signal over a serotype-specific and manually adjustable integration range;
(c5) establishing a calibration curve by applying non-linear regression to signals generated by calibration samples with known concentrations and thereby plotting the calculated area under the curve for each signal versus the concentration of the corresponding calibration sample;
(c6) calculating the concentration of the glycoconjugate within the test sample by comparing the measured area under the curve with the calibration curve;
(c7) evaluating the validity of the calculated concentration by automated comparison with pre-defined acceptance criteria.

In **step (c1),** the dataset generated in step (b) is received by a computer.

In **step (c2),** the background for each capillary is identified by applying a linear regression from the signal that was measured for each capillary before the start of the integration range to the signal that was measured after the end of the integration range.

Typically the skilled person would expect that identifying an average background signal over multiple capillaries or a reference background signal from one reference capillary and subtracting said reference or average background signal from each capillary is sufficient. However, it was surprisingly found that the accuracy of the results was improved when an individual background signal was identified for each capillary. Without wishing to be bound by theory, this may be due to an interference of the detectable signal generated in one capillary with the signal generated in the neighbouring capillaries.

This step is explained in further detail as follows:
In a **first step,** the limits for signal integration are set, thereby defining an integration range.

In a **second step,** the average from at least 3 data points, preferably all data points, that were measured within a first adjustable molecular weight window before the start of the integration range (average data point P1), and, at least 3 data points, preferably all data points, that were measured within a second adjustable molecular weight window (range for calculating the background signal) after the end of the integration range (average data point P2), are calculated.

### Molecular weight window:

The onset and the width of the first and the second window can be individually selected. As an example, the first window may span the range from X1 = "lowest X-axis value that is part of the integration range" to X2 = X1 - "width of the first adjustable molecular weight window [kDa]". The second window may span the range from X3 = "highest X-axis value that is part of the integration range" to X4 = X3 + "width of the second adjustable molecular weight window [kDa]".

A suitable width of the adjustable molecular weight window is 10 kDa. Nevertheless, the width of the molecular weight window may also be adjusted to deviate from a predefined value of 10 kDa, e.g. to 8 kDa or 13 kDa, as long as each, the first window and the second window, comprise at least 3 data points.

### Data points:

In one embodiment, the respective average data points P1 and P2 consist of the average of the signal intensities (y-axis) of at least 3 data points within the respective first or second molecular weight window and the average of the molecular weight at these data points (X-axis).

In another embodiment, the respective average data points P1 and P2 consist of the average of the signal intensities (y-axis) of at least 3 data points within the respective first or second molecular weight window and the molecular weight at the start or end of the integration range, respectively.

In a **third step,** linear regression is performed between the average data points P1 and P2 calculated above.

In **step (c3),** the background signal at each specific molecular weight, which is obtainable from the linear regression line calculated above, is subtracted from the corresponding total signal at each specific individual molecular weight, i.e. the difference between the signal detected within a capillary at each molecular weight and the background signal at the corresponding molecular weight.

In **step (c4),** the background corrected signal is integrated between the limits for integration set in (c2), first step. Said integration yields an area under the curve for each analyte. The Compass software included in the Wes^{™} system does not provide an option to receive limits for integration and to reliably integrate a whole signal area within these limits. It was surprisingly found that in case of glycoconjugates as described herein, in particular bioconjugates, the Compass software included in the Wes^{™} system fails to provide reliable integration of the whole signal area (cf. Fig. 1, 3, and Tables 3-5).

In **step (c5),** a calibration curve is established by applying non-linear regression to the signals generated by the calibration samples in step b). The calibration curve comprises the calculated area under the curve for each signal depending on the concentration of the corresponding calibration sample. Preferably, a bi-logarithmic with quadratic effects regression model is applied to establish the calibration curve, particularly according to the formula: ln(Area) = A ln(concentration)^2 + B ln (concentration) + C.

The Compass software included in the Wes^{™} system does not provide an option to establish a calibration curve based on such model but instead uses a linear regression model or a Four Parameter Logistic (4PL) Curve model. However, it was surprisingly found that establishing the calibration curve based on a bi-logarithmic with quadratic effects regression model improves the accuracy of the results.

In **step (c6),** the concentration of the glycoconjugate within the test sample is calculated by comparing the area under the curve that was measured for the test sample with the calibration curve.

In **step (c7),** the validity of the calculated concentration is evaluated by automated comparison with pre-defined acceptance criteria. Examples of pre-defined acceptance criteria are:
- Coefficient of determination (R²) for the calibration curve, for example R² ≥ 0.85, such as R² ≥ 0.90
- Coefficient of variation for calibration samples, for example CV ≤ 30%, such as CV ≤ 25%
- Coefficient of variation for the glycoconjugate within the test sample, for example CV ≤ 25%, such as CV ≤ 20%
- Coefficient of variation for the control sample within the test sample, for example CV ≤ 25%, such as CV ≤ 20%
- Relative difference of concentration calculated for the control sample to the known concentration of the control sample, for example
([calculated concentration] - [known concentration]) / [known concentration] ≤ 35%

Thus the aforementioned shortcoming is resolved by the combination of two features in the data analysis:

The first feature is the calculation of a background signal for each capillary and subtraction of said background signal from the signal which is generated by the glycoconjugate within that capillary. The background signal varies between the capillaries and thus reduces the reproducibility of quantification unless accounted for in each capillary.

The second feature is the possibility to receive limits for signal integration. These limits are typically set by the user. The thereby calculated concentration is automatically compared to pre-defined acceptance criteria. This allows to evaluate if the test sample complies with predefined acceptance criteria.

The following examples of the invention are to further illustrate the nature of the invention. It should be understood that the following examples do not limit the invention and the scope of the invention is to be determined by the appended claims.

### Example 1: Analysis of O-EPA bioconjugates according to the inventive method

### I. Preparation of reference material

A glycoprotein sample in larger quantities was directly obtained from a manufacturing batch and was aliquoted and stored appropriately (e.g. -80 °C). The different characterization steps were performed on thawed aliquots of said glycoprotein sample.

The test sample used was a glycoconjugate vaccine comprising 10 different glycoconjugates (each being a bioconjugate, the composition comprising the ten different bioconjugates being described for instance in WO 2020/191082), each comprising an EPA carrier protein with four glycosylation sites that is covalently linked to a PS component corresponding to one of the *E. coli* O-antigens of the serotypes O1A, O2, O4, O6A, O8, 015, 016, O18A, O25B or O75. Due to the presence of 4 glycosylation sites, each of the glycoconjugates was present as a mono-, di-, tri- or tetraglycosylated variant of the same serotype, or a mixture thereof. The following example refers to the identification and absolute quantification of an O-EPA glycoconjugate comprising a PS component corresponding to the *E*. *coli* serotype O4 present in the above-mentioned mixture of 10 different O-EPA bioconjugates but was likewise applicable to the other serotypes (Figure 1 relates to *E. coli* serotype O6, Figure 2 relates to *E. coli* serotype O4, Figure 3 relates to *E. coli* serotype O25B; Tables 2, 4 and 5 show results from glycoconjugate vaccine compositions comprising either nine or ten glycoconjugates corresponding to different *E. coli* serotypes).
**1) Determination of the correct identity of the reference material.** A standard western blot protocol was followed using a specific antibody which has been selected and tested for specificity for the polysaccharide chain of the glycoconjugate of interest. Optionally, the correct identity of the carrier protein is determined using a specific antibody for the carrier protein.
**2) Determination of the total polysaccharide content within the reference material.** A total acidic hydrolysis into monosaccharides was performed with subsequent analysis by ion chromatography and pulsed amperometric detection (IC-PAD), according to the following instructions.

An aliquot of the reference material was hydrolysed for 2 h at 120 °C using Trifluoracetic acid (TFA) at a final concentration of 1.8 M. Optimal hydrolysis conditions (temperature, TFA concentration and time) may vary depending on the starting concentration of the polysaccharides.

Optimal conditions must demonstrate to quantitatively release all the monosaccharides from the polysaccharide chain with no further degradation of the monosaccharide molecule targeted for absolute quantification.

After hydrolysis, the sample was cooled down to room temperature and then dried by using a SpeedVac at 30 °C overnight. The dry sample was completely resuspended in H₂O (MilliQ-grade) and transferred into an HPLC vial. A set of calibration standards was prepared using a commercially available monosaccharide (in this case N-Acetylglucosamine; GlcNAc). If the targeted monosaccharide for quantification undergoes modification during the above-mentioned hydrolysis step (e.g. N-Acetylglucosamine is turned into Glucosamine) the appropriate monosaccharide (e.g. Glucosamine) must be used, or alternatively, the above-mentioned hydrolysis procedure must also be performed on the set of calibration standards.

Then, an ion chromatography system (e.g. Dionex ICS-5000) equipped with a pulsed amperometric detector (PAD) and a disposable gold electrode on Polytetrafluoroethylene (PTFE) was prepared. A Dionex CarboPac PA1 analytical column (4x 250 mm) was used in combination with a Dionex CarboPac PA1 guard column (4x 50 mm). The system was equilibrated with Eluent A (16 mM NaOH for sample elution) and Eluent B (500 mM NaOH for column cleaning). The sample and the set of calibration standards were sequentially injected using the following instrument method / gradient profile:
- 0 to 24 min, 100 % Eluent A, 1 mL/min flow rate (elution)
- 25 to 32 min, 100 % Eluent B, 1 mL/min flow rate (wash)
- 33 to 60 min, 100 % Eluent A, 1 mL/min flow rate (reequilibrate)

Depending on the targeted monosaccharide, these gradients may need to be optimized.

The area under the curve from the measured calibration set was used to obtain a calibration curve and then the unknown sample was quantified. Optionally, the amount of targeted monosaccharide can also be used to back-calculate the absolute amount of repeating units/polysaccharides in µg/mL in the glycoconjugate sample.

### II. Analysis on the capillary-based immunoassay system

The test sample to be analysed (identification and absolute quantification) was directly obtained from a manufacturing batch of a glycoconjugate vaccine. A reference material was prepared as described above reflecting the same glycoconjugate species, i.e. the same PS component (in this case *E. coli* O4 antigen polysaccharide) and the same carrier protein (in this case detoxified EPA having SEQ ID NO: 3). As mentioned above, the test sample additionally comprised nine further O-EPA bioconjugates corresponding to *E. coli* serotypes O1A, O2, O6A, O8, 015, 016, O18A, O25B and O75.

The measurement was performed on the WES^{™} system (Bio-Techne) equipped with the software "Compass for SW version 3.1.7".

### 1. Adjustment of concentrations of the test sample, calibration samples and the control sample

The concentrations of the O4 O-EPA bioconjugate present within the test sample, the calibration samples and the control sample were diluted to the respective target concentrations using 0.1x sample buffer (SB) provided by Bio-Techne (Catalog # 042-195). For the test sample, the expected concentration was calculated from the concentrations that were measured for manufacturing batches of the individual glycoconjugates before the individual glycoconjugates were combined into a multivalent glycoconjugate vaccine composition.

For the bioconjugate comprising an EPA carrier protein with four glycosylation sites (SEQ ID NO: 3) covalently linked to a PS component corresponding to an *E. coli* O-antigen of the serotype O4, the following dilutions were performed:
(i) the calibration curve was generated from 4 calibration samples with different PS concentrations (0.200, 0.155, 0.110 and 0.065 µg/mL). Each calibration sample was prepared in triplicates;
(ii) the test sample, i.e. the glycoconjugate vaccine batch, was diluted to a PS concentration *(E. coli* O4 polysaccharide) of 0.150 µg/mL. The test sample was prepared in quadruplicates;
(iii) the control sample (SSC) was diluted to a PS concentration of 0.100 µg/mL. The control sample was prepared in triplicates.

Optimal dilutions for calibration samples and test sample must be evaluated for each glycoconjugate individually. The above dilutions were found to be optimal for identification and absolute quantification of O4 O-EPA bioconjugate.

### 2. Preparation of the fluorescent markers and a biotinylated ladder

The fluorescent markers and a biotinylated ladder were obtained from Bio-Techne (Catalog # PS-FL03-8). Two fluorescent markers were included in each capillary and were used to account for differences in the electrophoretic migration between individual capillaries. The fluorescent markers migrated at 57 kDa and 280 kDa. The biotinylated ladder comprised a multitude of proteins of a known molecular weight and was analysed in a separate capillary. The ladder was used as a size reference for the glycoconjugates to be analysed. The molecular weight of the proteins within the biotinylated ladder spanned the range from 66 kDa to 440 kDa. The fluorescent markers and the biotinylated ladder were prepared according to the manufacturer's instructions:
(i) the fluorescent markers were resuspended in 10X Sample buffer (Catalog # 042-195), supplemented with 400 mM DTT to create a 5X fluorescent master mix;
(ii) the biotinylated ladder was resuspended in deionized water.

### 3. Preparation of the test sample, calibration samples and the control samples for loading on an assay plate for the capillary-based immunoassay system

(i) 4.8 µL of each of the diluted test sample, calibration samples and control sample were each mixed with 1.2 µL of the 5X fluorescent master mix;
(ii) the mixtures were incubated at 95°C for 5 minutes;
(iii) the mixtures were cooled down by an incubation on ice for 5 minutes;
(iv) the mixtures were briefly vortexed, spun down, and 5 µL were loaded in separate compartments of an assay plate. One compartment of this assay plate was already pre-filled with Separation Matrix 3, Stacking Matrix 2, Split Running Buffer 3 and Matrix Removal Buffer (Bio-Techne, catalog# SM-W006). This compartment was already sealed by the vendor.

### 4. Preparation of the antibodies and substrates for loading on the assay plate

(i) a primary rat monoclonal antibody specific for the PS component of the O4-EPA glycoconjugate (*E*. *coli* O-antigen, serotype O4) was diluted in Antibody Diluent 2 (Bio-Techne, catalog# 042-203) to reach the final concentration of 0.05 mg/mL;
(ii) the secondary anti-rat-HRP antibody (Biolegend, catalog# 405405) was diluted with a 1:100 ratio in Antibody Diluent 2;
(iii) the substrate for capillary# 1 was prepared by mixing 15 µL of Luminol-S reagent and 15 µL of Peroxide reagent supplied in the detection module (Bio-Techne, catalog# DM-003);
(iv) the substrate for capillaries# 2-25 was prepared by mixing 220 µL of Luminol/Enhancer reagent and 220 µL of Peroxide reagent supplied in the Clarity^{™} Western ECL Substrate kit (BioRad, catalog# 170-5060);
(v) the antibodies (10 µL per well) and substrates (15 µL per well) were loaded on separate compartments of the assay plate prepared in step 3 (iv);
(vi) the washing buffer (Bio-Techne, catalog# 042-202) was added to separate compartments of the assay plate (500 µL per compartment) according to the manufacturer's instruction;
(vii) the assay plate was covered with a lid and centrifuged at 1100 x g for 5 minutes at room temperature to ensure that the liquid was at the bottom in all wells.

### 5. Loading of the assay plate on the WES^{™} system and starting the measurement

In this step, the assay plate obtained after step 4 (vii) was loaded on the WES^{™} equipment (Bio-Techne) to start the analysis:
(i) the capillary western system and its related computer were switched on. After the light signal of the instrument stopped pulsing, the system was ready to be used and the Compass software which was provided with the WES^{™} system was started;
(ii) the "new run" window was opened in the Compass software and the following parameters were selected: "Size" as Assay Type, "66-440 kDa" as Size Range, and "25" as Cartridge;
(iii) the capillary cartridge (Bio-Techne, catalog# SM-W006) was inserted into the appropriate holder and the interior light changed from orange to blue, indicating that the cartridge was properly inserted;
(iv) after removal of the assay plate lid, the evaporation seal was carefully peeled off from the plate by firmly holding it on the bench; if bubbles are present in the Separation Matrix wells such bubbles can be removed using a clean needle (was not necessary in this case);
(v) the pre-filled assay plate was placed on the plate holder, the instrument door was closed and the assay run was started from the Compass software.

### 6. Data analysis

(i) In the Compass software, the correct position of the fluorescent markers (57 and 280 kDa) were verified: if the signals corresponding to the fluorescent markers are wrongly assigned by the software, the position of the signal can be corrected to 57 and 280 kDa, respectively, by selecting "Not a standard" or "Force Standard" (was not necessary in this case);
(ii) the datasets were exported from the Compass Software by selecting: File → Export Spectra → Text Format (a new folder containing the spectra was automatically created in the same folder where the run has been saved);
(iii) the dataset (Sample Plots Raw.txt) was imported into the computer program of the inventive method to analyse the data;
(iv) the sample ID, the serotype, the instrument used and the date of the analysis were entered;
(v) the integration range (100 - 480 kDa) and relative baseline evaluation range (molecular weight window: 10 kDa; 90 - 100 kDa and 480 - 490 kDa) were entered;
(vi) a calibration curve was established by applying to the signals generated by the calibration samples a bi-logarithmic with quadratic effects regression model according to the formula: ln(Area) = A ln(concentration)^2 + B ln(concentration) + C. The calibration curve comprised the calculated area under the curve for each signal depending on the concentration of the corresponding calibration sample. The concentration of the glycoconjugate within the test sample was calculated by comparing the area under the curve that was measured for the test sample with the calibration curve. This comparison was made by inversion of the above-mentioned regression function underlying the calibration curve. Said inversion of the above-mentioned regression function yields 2 results. The most probable result, i.e. the result that is closer to the expected content of the glycoconjugate within the test sample, is selected.

Step (v): A capillary-specific background signal was calculated by the computer program and subtracted from the total signal that was measured within this capillary.

In analogy to the above, the concentrations of the other nine bioconjugates present within the glycoconjugate vaccine (each bioconjugate comprising detoxified EPA having SEQ ID NO: 3 covalently linked to one of *E. coli* O-antigen polysaccharides of the serotypes O1A, O2, O6A, O8, 015, 016, O18A, O25B or O75) were determined using the corresponding antibodies.

The results of this specific example were as follows (taken into account the dilution in step II, 3):

**Table 2: Results from the analysis of a glycoconjugate vaccine composition comprising ten different O-EPA bioconjugates (E. coli O-polysaccharides) using the inventive method**

| *E. coli* serotype | Measured PS Content [µg/ml] | Target PS content [µg/ml] |
|---|---|---|
| O1A | 8.22 | 8 |
| O2 | 7.61 | 8 |
| O4 | 6.49 | 8 |
| O6A | 8.59 | 8 |
| O8 | 6.40 | 8 |
| O15 | 8.23 | 8 |
| O16 | 8.36 | 8 |
| O18A | 6.28 | 8 |
| O25B | 15.87 | 16 |
| O75 | 7.38 | 8 |

### Example 2: Comparative analysis of O25BO-EPA bioconjugate according to prior art and inventive method

Two batches of a glycoconjugate vaccine composition comprising 9 different bioconjugates (each bioconjugate comprising detoxified EPA having SEQ ID NO: 3 covalently linked to one of *E. coli* O-antigen polysaccharides of the serotypes O1A, O2, O4, O6A, 015, 016, O18A, O25B, and O75) were analyzed with respect to the O25B O-EPA bioconjugate concentration according to the prior art (Wes^{™} with Compass Software) and the inventive method.

A calibration curve was established based on 4 O25B glycoconjugate calibration samples (0.080, 0.063, 0.045, 0.028 µg/mL; concentration based on PS component) according to the procedure described above (parallel analysis in different capillaries of the Wes^{™} system; dilution as was found optimal for O25B O-EPA bioconjugate). Each of the above calibration samples was measured in independently prepared triplicates (replicates denoted as a-c in Table 3). The whole signal area for each sample was analyzed using both the commercially available Compass software (prior art) and the computer program described herein (inventive method). The resulting AUCs according to both methods and the coefficient of variation (CV) based on the triplicate measurements are shown in Table 3 below. Electropherograms corresponding to triplicates of the 0.063 µg/ml O25B O-EPA calibration sample are shown in Fig. 3 (A-C: integration via Compass software; D-F: integration via computer program as described herein).

It can be seen from both Fig. 3 and Table 3 that
(i) AUC according to the prior art method is lower than AUC according to the inventive method due incomplete integration of the whole signal area; and
(ii) Lower precision of analysis according to prior art (higher CV); higher deviation of individual measurements including occasional failure to integrate the signal (lower reproducibility).

**Table 3: O25B O-EPA bioconjugate calibration samples analyzed according to prior art and inventive method**

| Calibration sample | AUC prior art | CV prior art | AUC inventive method | CV inventive method |
|---|---|---|---|---|
| O25B-EPA_0.080 (a) | 1901817 | 58 | 3504249 | 12 |
| O25B-EPA_0.063 (a) | 0 | 88 | 2869471 | 17 |
| O25B-EPA_0.045 (a) | 896673 | 87 | 1676823 | 13 |
| O25B-EPA_0.028 (a) | 516343 | 15 | 912080 | 18 |
| O25B-EPA_0.080 (b) | 598843 | - | 3068881 | - |
| O25B-EPA_0.063 (b) | 903532 | - | 2334112 | - |
| O25B-EPA_0.045 (b) | 961544 | - | 1755261 | - |
| O25B-EPA_0.028 (b) | 678355 | - | 1223598 | - |
| O25B-EPA_0.080 (c) | 972741 | - | 2770255 | - |
| O25B-EPA_0.063 (c) | 1074113 | - | 2067236 | - |
| O25B-EPA_0.045 (c) | 0 | - | 2138194 | - |
| O25B-EPA_0.028 (c) | 679008 | - | 1310229 | - |

The standard curve was established based on the results shown in Table 3, either according to
a) the linear model provided in the Compass software (alternatively provided in the Compass software is a "Four Parameter Logistic Model" but which was not suitable for the dataset); or
b) a bi-logarithmic with quadratic effects regression model according to formula: ln(Area) = A ln(concentration)^2 + B ln(concentration) + C (inventive method) .

Then, two batches of the above-mentioned glycoconjugate vaccine composition comprising 9 different bioconjugates (each bioconjugate comprising detoxified EPA having SEQ ID NO: 3 covalently linked to one of *E. coli* O-antigen polysaccharides of the serotypes O1A, O2, O4, O6A, 015, 016, O18A, O25B, and O75) were measured in quadruplicates on the Wes^{™} system as described above (replicates denoted as a-d in Table 4). Test samples were diluted to an O25B O-EPA target concentration of 0.063 µg/ml based on the *E. coli* O25B PS component. Whole signal integration was performed either using the Compass software (prior art) or using the computer program described herein.

Then, absolute quantification of O25B O-EPA bioconjugate present within the above-described 9-valent glycoconjugate vaccine composition was performed based on
a) the above-described calibration curve obtained using the Compass software (prior art); or
b) the above-described calibration curve obtained using the computer program described herein (inventive method) .

In analogy to the above, an O25B O-EPA bioconjugate control sample (system suitability control; SSC) was measured in parallel (separate capillaries) and analyzed according to the prior art method or the inventive method.

Results (taken into account the dilution) are shown in Table 4.

**Table 4: O25B O-EPA bioconjugate present within a 9-valent glycoconjugate vaccine composition (E. coli serotypes O1A, O2, O4, O6A, 015, 016, O18A, O25B, and O75) analyzed according to prior art and inventive method**

| Sample | Target PS content [µg/ml] | Measured PS Content [µg/ml] | Measured PS Content [µg/ml] |
|---|---|---|---|
| | | prior art | Inventive method |
| O25B-EPA (batch 1_a) | 32 | 55.87 | 32.19 |
| O25B-EPA (batch 1_b) | 32 | 56.53 | 32.59 |
| O25B-EPA (batch 1_c) | 32 | 6.61 | 26.13 |
| O25B-EPA (batch 1_d) | 32 | 58.51 | 34.28 |
| O25B-EPA (batch 2_a) | 32 | 10.13 | 35.02 |
| O25B-EPA (batch 2_b) | 32 | 66.38 | 37.76 |
| O25B-EPA (batch 2_c) | 32 | 12.66 | 38.00 |
| O25B-EPA (batch 2_d) | 32 | 71.26 | 41.19 |
| SSC_a | 349 | 557.27 | 348.80 |
| SSC b | 349 | 681.83 | 414.59 |
| SSC_c | 349 | 593.41 | 366.79 |

It can be seen from Table 4 that the method of the prior art (Compass software) failed to provide reliable absolute quantification of the O25B O-EPA bioconjugate for both batches of the glycoconjugate vaccine composition and the control sample, whereas the inventive method provided accurate data on both batches of the test sample and on the control sample.

In analogy to the above, also the other O-EPA bioconjugates present within batch 1 of the above-described 9-valent glycoconjugate vaccine composition were analyzed according to the prior art (Compass software) and the inventive method. Results (mean of independent triplicates) are shown in Table 5 below.

**Table 5: Comparative results from the analysis of a glycoconjugate vaccine composition comprising nine different O-EPA bioconjugates (E. coli O-polysaccharides) using the inventive method or according to the prior art**

| *E. coli* serotype | Target PS content [µg/ml] | Measured PS content [µg/ml] | Measured PS content [ug/ml] |
|---|---|---|---|
| | | prior art | inventive method |
| O1A | 16 | 14.86 | 14.70 |
| O2 | 16 | 16.16 | 16.08 |
| O4 | 16 | 16.98 | 15.68 |
| O6A | 16 | 19.95 | 17.27 |
| 015 | 16 | 16.28 | 16.63 |
| 016 | 16 | 18.24 | 18.99 |
| O18A | 16 | 2.80 | 15.20 |
| O25B | 32 | 44.19 | 31.27 |
| O75 | 32 | 33.77 | 33.25 |

Whereas analysis according to the prior art may provide accurate data on some of the O-EPA bioconjugates present in the 9-valent glycoconjugate composition, the method of the prior art fails to provide reliable absolute quantification on the other bioconjugates (e.g. O6A, O18A and O25B O-EPA bioconjugates) . In some cases (O18A and O25B O-EPA bioconjugates), the Compass software in fact completely fails to provide meaningful data.

However, the inventive method provided reliable absolute quantification on all bioconjugates, including the particularly challenging O18A and O25B O-EPA bioconjugates. Identification of the bioconjugates was successfully achieved through the generation of a detectable signal upon binding of a PS-specific antibody to each of the bioconjugates.

### Description of the sequences:

**SEQ ID NO: 1** (glycosylation consensus sequence) Asn-X-Ser(Thr), wherein X can be any amino acid except Pro
**SEQ ID NO: 2** (optimized glycosylation consensus sequence) Asp(Glu)-X-Asn-Z-Ser(Thr), wherein X and Z are independently selected from any amino acid except Pro
**SEQ ID NO: 3** (EPA carrier protein including 4 N-linked glycosylation consensus sequences)

GSGGGDQNATGSGGGKLAEEAFDLWNECAKACVLDLKDGVRSSRMSVDPAIADTNGQGVLHYSMVLEG
GNDALKLAIDNALSITSDGLTIRLEGGVEPNKPVRYSYTRQARGSWSLNWLVPIGHEKPSNIKVFIHE
LNAGNQLSHMSPIYTIEMGDELLAKLARDATFFVRAHESNEMQPTLAISHAGVSVVMAQAQPRREKRW
SEWASGKVLCLLDPLDGVYNYLAQQRCNLDDTWEGKIYRVLAGNPAKHDLDIKDNNNSTPTVISHRLH
FPEGGSLAALTAHQACHLPLEAFTRHRQPRGWEQLEQCGYPVQRLVALYLAARLSWNQVDQVIRNALA
SPGSGGDLGEAIREQPEQARLALTLAAAESERFVRQGTGNDEAGAASADVVSLTCPVAKDQNRTKGEC
AGPADSGDALLERNYPTGAEFLGDGGDVSFSTRGTQNWTVERLLQAHRQLEERGYVFVGYHGTFLEAA
QSIVFGGVRARSQDLDAIWRGFYIAGDPALAYGYAQDQEPDARGRIRNGALLRVYVPRWSLPGFYRTG
LTLAAPEAAGEVERLIGHPLPLRLDAITGPEEEGGRVTILGWPLAERTVVIPSAIPTDPRNVGGDLDP
SSIPDKEQAISALPDYASQPGKPPREDLKLGSGGGDQNAT

## Claims

1. A method for analysing a glycoconjugate in a test sample which comprises a mixture of at least 4 glycoconjugates,
wherein the analysis includes:
• the identification of said glycoconjugate and
• the absolute quantification of said glycoconjugate based on a calibration curve;
wherein the method comprises the steps of:
(a) providing the test sample and a set of calibration samples, optionally a ladder sample, and optionally a control sample;
(b) measuring, by means of a capillary-based immunoassay system, in individual capillaries: said test sample, said calibration samples, optionally said ladder sample, and optionally said control sample, thereby generating a dataset for each capillary; and
(c) analysing said dataset by means of a computer program which provides at least the following functions:
• receiving limits for integration; and
• calculating a background signal for each capillary; and
• subtracting a background signal from a signal generated in each capillary.

2. The method according to claim 1 wherein measuring of said glycoconjugate leads to a broad signal, in particular a broad signal comprising not-fully resolved peaks.

3. The method according to claim 1 or 2, wherein said glycoconjugates are components of a glycoconjugate vaccine.

4. The method according to any one of claims 1 - 3, wherein said glycoconjugate is a bioconjugate, in particular a bioconjugate which is produced by enzymatic conjugation of a polysaccharide component to a carrier protein, preferably using a PglB oligosaccharyltransferase system in *E. coli.*

5. The method according to any one of claims 1 - 4, wherein said glycoconjugate comprises one carrier protein and one or more polysaccharide(s) covalently bound to said carrier protein wherein
(i) said carrier protein is a detoxified Exotoxin A of *Pseudomonas aeruginosa* (EPA) and
(ii) said polysaccharide is an *Escherichia coli* O-antigen selected from the group consisting of O1A, O2, O4, O6A, O8, O15, O16, O18A, O25B or O75.

6. The method according to any one of claims 1 - 5, wherein said polysaccharide(s) comprise 1-100, preferably 3-30, more preferably 5 - 20, repeating units, said repeating units comprising non-modified monosaccharides and/or modified monosaccharides, the modified monosaccharides being in particular O-acetylated and/or N-acetylated monosaccharides.

7. The method according to any one of claims 1 - 6, wherein said test sample further comprises:
• an aqueous matrix, said matrix optionally including one or more of buffers, inorganic salts, sugar alcohols, and/or non-ionic surfactants;
• optionally polysaccharides not bound to carrier protein ("free PS");
• optionally non-related proteins.

8. The method according to any one of claims 1 - 7, wherein said test sample comprises a multitude of different glycoconjugates, preferably 4 - 20 glycoconjugates, preferably 4-10 glyconjugates, such as 4, 9 or 10 glycoconjugates, said glycoconjugates differing in the polysaccharide components listed in claim 5 (ii).

9. The method according to any one of claims 1 - 8, wherein said analysis includes
• the identification of mono-, di-, tri- and/or tetraglycosylated variants of said glycoconjugate and/or
• the absolute quantification of mono-, di-, tri- and/or tetraglycosylated variants of said glycoconjugate based on a calibration curve.

10. The method according to any one of claims 1 - 9, wherein said step a) further comprises one or more of the following steps, preferably in the order as indicated:
(a1) adjusting the concentration of the glycoconjugate to an expected concentration of 0.01 - 0.50 µg mL⁻¹;
(a2) adding to the sample one or more auxiliary reagents selected from a sample buffer, a disulfide bridge reducing agent and one or more markers;
(a3) denaturing the sample, preferably by applying heat.

11. The method according to any one of claims 1 - 10, wherein said step b) further comprises one or more of the following steps, preferably in the order as indicated:
(b1) loading an analysis matrix, preferably comprising a size-exclusion matrix, into the capillaries of said immunoassay system;
(b2) loading the test sample, calibration samples, optional ladder sample, and optional control sample into individual capillaries of said immunoassay system;
(b3) separating the components of said samples;
(b4) immobilising the components of said sample;
(b5) applying a glycan-specific primary antibody which binds to said glycoconjugate;
(b6) applying a secondary antibody which binds to the primary antibody and which generates a detectable signal;
(b7) in case the secondary antibody is linked to an enzyme, applying a substrate for said enzyme.

12. The method according to claim 11, wherein in step (b6) the detectable signal is generated in that said secondary antibody is covalently linked to an enzyme, such as horseradish peroxidase, capable of catalyzing a chemiluminescent reaction, a chemifluorescent reaction or a chemical reaction leading to a colored or fluorescent product.

13. The method according to any one of claims 1 - 12, wherein step c) further comprises one or more of the following steps, preferably in the order as indicated:
(c1) receiving the dataset for every capillary generated in step b);
(c2) identifying the background for every capillary;
(c3) subtracting the individual background signal from the measured signal of the corresponding capillary;
(c4) obtaining an area under the curve for every capillary by integrating the background corrected signal over a polysaccharide-specific and adjustable integration range;
(c5) establishing a calibration curve by applying non-linear regression, preferably by applying a bi-logarithmic with quadratic effects regression model, to signals generated by calibration samples with known concentrations and thereby plotting the calculated area under the curve for each signal versus the concentration of the corresponding calibration sample;
(c6) calculating the concentration of the glycoconjugate within the test sample by comparing the measured area under the curve with the calibration curve; and
(c7) evaluating the validity of the calculated concentration by automated comparison with pre-defined acceptance criteria.

## Patentansprüche

1. Verfahren zum Analysieren eines Glykokonjugats in einer Testprobe, die ein Gemisch von wenigstens 4 Glykokonjugaten umfasst,
wobei die Analyse beinhaltet:
• die Identifizierung des Glykokonjugats und
• die absolute Quantifizierung des Glykokonjugats auf Grundlage einer Kalibrierungskurve;
wobei das Verfahren die Schritte umfasst:
(a) Bereitstellen der Testprobe und eines Satzes von Kalibrierungsproben, optional einer Leiterprobe und optional einer Kontrollprobe;
(b) Messen mittels eines kapillarbasierten Immunoassaysystems in individuellen Kapillaren: der Testprobe, der Kalibrierungsproben, optional der Leiterprobe und optional der Kontrollprobe, wobei dadurch ein Datensatz für jede Kapillare erzeugt wird; und
(c) Analysieren des Datensatzes mittels eines Computerprogramms, das wenigstens die folgenden Funktionen bereitstellt:
• Empfangen von Grenzwerten für die Integration; und
• Berechnen eines Hintergrundsignals für jede Kapillare; und
• Subtrahieren eines Hintergrundsignals von einem in jeder Kapillare erzeugten Signal.

2. Verfahren nach Anspruch 1, wobei das Messen des Glykokonjugats zu einem breiten Signal führt, insbesondere einem breiten Signal, das nicht vollständig aufgelöste Peaks umfasst.

3. Verfahren nach Anspruch 1 oder 2, wobei die Glykokonjugate Komponenten eines Glykokonjugatimpfstoffs sind.

4. Verfahren nach einem der Ansprüche 1 - 3, wobei das Glykokonjugat ein Biokonjugat ist, insbesondere ein Biokonjugat, das durch enzymatische Konjugation einer Polysaccharidkomponente an ein Trägerprotein hergestellt wird, vorzugsweise unter Verwendung eines PglB-Oligosaccharyltransferase-Systems in *E. coli.*

5. Verfahren nach einem der Ansprüche 1 - 4, wobei das Glykokonjugat ein Trägerprotein und ein oder mehrere Polysaccharide umfasst, die kovalent an das Trägerprotein gebunden sind, wobei
(i) das Trägerprotein ein entgiftetes Exotoxin A von *Pseudomonas aeruginosa* (EPA) ist und
(ii) das Polysaccharid ein *Escherichia coli* O-Antigen ist, ausgewählt aus der Gruppe bestehend aus O1A, O2, O4, O6A, O8, 015, 016, 018A, O25B oder O75.

6. Verfahren nach einem der Ansprüche 1 - 5, wobei das/die Polysaccharid(e) 1-100, vorzugsweise 3-30, stärker bevorzugt 5-20 sich wiederholende Einheiten umfasst/umfassen, wobei die sich wiederholenden Einheiten nicht modifizierte Monosaccharide und/oder modifizierte Monosaccharide umfassen, wobei die modifizierten Monosaccharide insbesondere O-acetylierte und/oder N-acetylierte Monosaccharide sind.

7. Verfahren nach einem der Ansprüche 1 - 6, wobei die Testprobe ferner umfasst:
• eine wässrige Matrix, wobei die Matrix optional einen oder mehrere Puffer, anorganische Salze, Zuckeralkohole und/oder nichtionische Tenside beinhaltet;
• optional Polysaccharide, die nicht an ein Trägerprotein ("freies PS"); gebunden sind;
• optional nicht verwandte Proteine.

8. Verfahren nach einem der Ansprüche 1 - 7, wobei die Testprobe eine Mehrzahl von verschiedenen Glykokonjugaten umfasst, vorzugsweise 4-20 Glykokonjugaten, vorzugsweise 4-10 Glykokonjugaten, wie 4, 9 oder 10 Glykokonjugate, wobei sich die Glykokonjugate in den Polysaccharidkomponenten, wie in Anspruch 5 (ii) aufgeführt, unterscheiden.

9. Verfahren nach einem der Ansprüche 1 - 8, wobei die Analyse beinhaltet:
• die Identifizierung von mono-, di-, tri- und/oder tetraglykosylierten Varianten des Glykokonjugats und/oder
• die absolute Quantifizierung von mono-, di-, tri- und/oder tetraglykosylierten Varianten des Glykokonjugats auf Grundlage einer Kalibrierungskurve.

10. Verfahren nach einem der Ansprüche 1 - 9, wobei der Schritt a) ferner einen oder mehrere der folgenden Schritte umfasst, vorzugsweise in der angegebenen Reihenfolge:
(a1) Anpassen der Konzentration des Glykokonjugats auf eine erwartete Konzentration von 0,01-0,50 µg mL-1;
(a2) Zugeben eines oder mehrerer Hilfsmittel zu der Probe, ausgewählt aus einem Probenpuffer, einem Disulfidbrückenreduktionsmittel und einem oder mehreren Markern;
(a3) Denaturieren der Probe, vorzugsweise durch Anwenden von Wärme.

11. Verfahren nach einem der Ansprüche 1 - 10, wobei der Schritt b) ferner einen oder mehrere der folgenden Schritte umfasst, vorzugsweise in der angegebenen Reihenfolge:
(b1) Laden einer Analysematrix, die vorzugsweise eine Größenausschlussmatrix umfasst, in die Kapillare des Immunoassaysystems;
(b2) Laden der Testprobe, der Kalibrierungsproben, der optionalen Leiterprobe und der optionalen Kontrollprobe in einzelne Kapillare des Immunoassaysystems;
(b3) Trennen der Komponenten der Proben;
(b4) Immobilisieren der Komponenten der Probe;
(b5) Anwenden eines glykanspezifischen primären Antikörpers, der sich an das Glykokonjugat bindet;
(b6) Anwenden eines sekundären Antikörpers, der sich an den primären Antikörper bindet und der ein nachweisbares Signal erzeugt;
(b7) falls der sekundäre Antikörper an ein Enzym gebunden ist, Anwenden eines Substrats für dieses Enzym.

12. Verfahren nach Anspruch 11, wobei in Schritt (b6) das nachweisbare Signal erzeugt wird, indem der sekundäre Antikörper kovalent an ein Enzym, wie Meerrettichperoxidase, gebunden ist, das in der Lage ist, eine Chemilumineszenzreaktion, eine Chemifluoreszenzreaktion oder eine chemische Reaktion zu katalysieren, die zu einem farbigen oder fluoreszierenden Produkt führt.

13. Verfahren nach einem der Ansprüche 1 - 12, wobei Schritt c) ferner einen oder mehrere der folgenden Schritte umfasst, vorzugsweise in der angegebenen Reihenfolge:
(c1) Empfangen des Datensatzes für jede Kapillare, die in Schritt b) erzeugt wurde;
(c2) Identifizieren des Hintergrunds für jede Kapillare;
(c3) Subtrahieren des individuellen Hintergrundsignals von dem gemessenen Signal der entsprechenden Kapillare;
(c4) Erhalten einer Fläche unter der Kurve für jede Kapillare durch Integrieren des hintergrundkorrigierten Signals über einen polysaccharidspezifischen und anpassparen Integrationsbereich;
(c5) Erstellen einer Kalibrierkurve durch Anwenden einer nichtlinearen Regression, vorzugsweise durch Anwenden eines bi-logarithmischen Regressionsmodells mit quadratischen Effekten, auf Signale, die von Kalibrierungsproben mit bekannten Konzentrationen erzeugt wurden, und dadurch Aufzeichnen der berechneten Fläche unter der Kurve für jedes Signal gegenüber der Konzentration der entsprechenden Kalibrierungsprobe;
(c6) Berechnen der Konzentration des Glykokonjugats innerhalb der Testprobe durch Vergleichen der gemessenen Fläche unter der Kurve mit der Kalibrierungskurve; und
(c7) Bewerten der Gültigkeit der berechneten Konzentration durch automatisierten Vergleich mit vordefinierten Akzeptanzkriterien.

## Revendications

1. Procédé pour analyser un glycoconjugué dans une éprouvette qui comprend un mélange d'au moins 4 glycoconjugués, dans lequel l'analyse inclut :
• l'identification dudit glycoconjugué, et
• la quantification absolue dudit glycoconjugué, sur la base d'une courbe d'étalonnage ;
dans lequel le procédé comprend les étapes de :
(a) la fourniture de l'éprouvette et d'un jeu d'échantillons d'étalonnage, facultativement d'un échantillon d'échelle, et facultativement d'un échantillon de contrôle ;
(b) la mesure, au moyen d'un système d'immuno-essai à base de capillaires, dans des capillaires individuels : de ladite éprouvette, desdits échantillons d'étalonnage, facultativement dudit échantillon d'échelle, et facultativement dudit échantillon de contrôle, ainsi générant un jeu de données pour chaque capillaire ; et
(c) l'analyse dudit jeu de données au moyen d'un programme informatique qui fournit au moins les fonctions suivantes :
• la réception de limites pour intégration ; et
• le calcul d'un signal de fond pour chaque capillaire ; et
• la soustraction d'un signal de fond à partir d'un signal généré dans chaque capillaire.

2. Procédé selon la revendication 1, dans lequel la mesure dudit glycoconjugué a pour résultat un signal général, en particulier un signal général comprenant des crêtes non entièrement résolues.

3. Procédé selon la revendication 1 ou 2, dans lequel lesdits glycoconjugués sont des composants d'un vaccin glycoconjugué.

4. Procédé selon l'une quelconque des revendications 1 à 3, dans lequel ledit glycoconjugué est un bioconjugué, en particulier un bioconjugué qui est produit par conjugaison enzymatique d'un composant polysaccharide avec une protéine-support, de préférence en utilisant un système de oligosaccharyltransférase PglB dans *E. coli.*

5. Procédé selon l'une quelconque des revendications 1 à 4, dans lequel ledit glycoconjugué comprend une protéine-support et un ou plusieurs polysaccharide(s) lié(s) de façon covalente à ladite protéine-support, dans lequel
(i) ladite protéine-support est une exotoxine A détoxifiée de *Pseudomonas aeruginosa* (EPA) et
(ii) ledit polysaccharide est un antigène O d*'Escherichia coli* sélectionné parmi le groupe constitué de : O1A, O2, O4, O6, O8, 015, 016, O18A, O25B, ou O75.

6. Procédé selon l'une quelconque des revendications 1 à 5, dans lequel ledit (lesdits) polysaccharide(s) comprend (comprennent) de 1 à 100, de préférence de 3 à 30, mieux encore de 5 à 20, unités répétées, lesdites unités répétées comprenant des monosaccharides non modifiés et/ou des monosaccharides modifiés, les monosaccharides modifiés étant en particulier des monosaccharides O-acétylés et/ou N-acétylés.

7. Procédé selon l'une quelconque des revendications 1 à 6, dans lequel ladite éprouvette comprend en outre :
• une matrice aqueuse, ladite matrice incluant facultativement un ou plusieurs de : tampons, sels inorganiques, alcools glucidique, et/ou surfactants non ioniques ;
• facultativement des polysaccharides non liés à protéine-support (« PS libres ») ;
• facultativement des protéines non connexes.

8. Procédé selon l'une quelconque des revendications 1 à 7, dans lequel ladite éprouvette comprend une multitude de différents glycoconjugués, de préférence de 4 à 20 glycoconjugués, de préférence de 4 à 10 glyconjugués, tels que 4, 9 ou 10 glycoconjugués, lesdits glycoconjugués étant différents en composants polysaccharides listés dans la revendication 5 (ii).

9. Procédé selon l'une quelconque des revendications 1 à 8, dans lequel ladite analyse inclut
• l'identification de variants mono-, di-, tri- et/ou tétra-glycosylés dudit glycoconjugué, et/ou
• la quantification absolue de variants mono-, di-, tri- et/ou tétra-glycosylés dudit glycoconjugué, sur la base d'une courbe d'étalonnage.

10. Procédé selon l'une quelconque des revendications 1 à 9, dans lequel ladite étape a) comprend en outre une ou plusieurs des étapes suivantes, de préférence dans l'ordre tel qu'indiqué :
(a1) l'ajustement de la concentration du glycoconjugué à une concentration escomptée de 0,01 à 0,50 µg mL⁻¹ ;
(a2) l'ajout, à l'échantillon, d'un ou de plusieurs réactifs auxiliaires sélectionnés parmi un tampon d'échantillon, un agent réducteur à pont disulfure, et un ou plusieurs marqueurs ;
(a3) la dénaturation de l'échantillon, de préférence en appliquant de la chaleur.

11. Procédé selon l'une quelconque des revendications 1 à 10, dans lequel ladite étape b) comprend en outre une ou plusieurs des étapes suivantes, de préférence dans l'ordre tel qu'indiqué :
(b1) le chargement d'une matrice d'analyse, de préférence comprenant une matrice d'exclusion de tailles, dans les capillaires dudit système d'immuno-essai ;
(b2) le chargement de l'éprouvette, des échantillons d'étalonnage, de l'échantillon d'échelle facultatif, et de l'échantillon de contrôle facultatif dans des capillaires individuels dudit système d'immuno-essai ;
(b3) la séparation des composants desdits échantillons ;
(b4) l'immobilisation des composants dudit échantillon ;
(b5) l'application d'un anticorps primaire spécifique de glycane qui se lie audit glycoconjugué ;
(b6) l'application d'un anticorps secondaire qui se lie à l'anticorps primaire et qui génère un signal détectable ;
(b7) au cas où l'anticorps secondaire serait lié à un enzyme, l'application d'un substrat pour ledit enzyme.

12. Procédé selon la revendication 11, dans lequel, dans l'étape (b6), le signal détectable est généré, en ce que ledit anticorps secondaire est lié de façon covalente à un enzyme, tel que de la peroxydase de raifort, capable de catalyser une réaction chimioluminescente, un réaction chimiofluorescente, ou une réaction chimique ayant pour résultat un produit coloré ou fluorescent.

13. Procédé selon l'une quelconque des revendications 1 à 12, dans lequel l'étape c) comprend en outre une ou plusieurs des étapes suivantes, de préférence dans l'ordre tel qu'indiqué :
(c1)la réception du jeu de données pour chaque capillaire, généré dans l'étape b) ;
(c2) l'identification du fond pour chaque capillaire ;
(c3) la soustraction du signal de fond individuel, du signal mesuré du capillaire correspondant ;
(c4) l'obtention d'une aire sous la courbe pour chaque capillaire en intégrant le signal corrigé de fond sur une plage d'intégration ajustable et spécifique de polysaccharide ;
(c5) l'établissement d'une courbe d'étalonnage en appliquant une régression non-linéaire, de préférence en appliquant un modèle de régression bi-logarithmique avec effets quadratiques, à des signaux générés par des échantillons d'étalonnage avec des concentrations connues, et ainsi la représentation graphique de l'aire calculée sous la courbe pour chaque signal par rapport à la concentration de l'échantillon d'étalonnage correspondant ;
(c6) le calcul de la concentration du glycoconjugué à l'intérieur de l'éprouvette en comparant l'aire mesurée, sous la courbe, à la courbe d'étalonnage ; et
(c7) l'évaluation de la validité de la concentration calculée par comparaison automatisée à des critères d'acceptation prédéfinis.
